# EUROPÄISCHE PATENTSCHRIFT

(11) **EP 1 408 943 B1**
(45) Veröffentlichungstag und Bekanntmachung des Hinweises auf die Patenterteilung: **29.06.2005**
(21) Anmeldenummer: 02787119.3
(22) Anmeldetag: 09.07.2002
(51) Int. Cl.: A61K 31/155, A61K 9/20, A61P 1/00, A61P 17/06, A61P 25/28, A61P 11/06, A61P 19/02

(54) **ARZNEIMITTELFORMULIERUNG ENTHALTEND EINEN LTB4 ANTAGONISTEN UND EINEN NETZMITTEL**
PHARMACEUTICAL FORMULATION CONTAINING AN LTB4 ANTAGONIST AND A WETTING AGENT
FORMULATION MEDICAMENTEUSE CONTENANT UN ANTAGONISTE DU LTB 4 ET UN AGENT MOUILLANT

(30) Priorität: 14.07.2001 DE 10134377; 15.02.2002 DE 10206241
(43) Veröffentlichungstag der Anmeldung: 21.04.2004
(73) Patentinhaber: Boehringer Ingelheim Pharma GmbH & Co.KG, 55216 Ingelheim am Rhein (DE)
(72) Erfinder: BOCK, Thomas, 2300 Copenhagen S (DK); BECKER, Robert, 88400 BIBERACH/RISS (DE); SAUER, Achim, 88214 RAVENSBURG-TORKENWEILER (DE); ROSCHER, Rene, 78464 KONSTANZ (DE); ROTH, Ulrich, 88400 BIBERACH (DE); MOLL, Siglinde, 88437 ÄPFINGEN GDE. MASELHEIM (DE); WEBER, Karl, 88456 Grodt/Ingoldingen (DE); KOHLRAUSCH, Anja, 88400 BIBERACH (DE)
(86) Internationale Anmeldenummer: PCT/EP2002/007618
(87) Internationale Veröffentlichungsnummer: WO 2003/007922

(56) Entgegenhaltungen:
- WO-A-01/25186
- WO-A-93/16036
- WO-A-94/11341
- WO-A-96/02497
- WO-A-97/21670
- WO-A-98/11062
- WO-A-98/11119
- HUNNIUS: "PHARMAZEUTISCHES WÖRTERBUCH" 1993 , DE GRUYTER , BERLIN / NEW YORK XP002228284 "ZERFALLMITTEL" Seite 1520 -Seite 1521
- ARTHUR H KIBBE: "HANDBOOK OF PHARMACEUTICAL EXCIPIENTS" 2000 , AMERICAL PHARMACEUTICAL ASSOCIATION , WASHINTON, USA XP002228285 Seite 487-489

## Beschreibung

Die Erfindung betrifft eine neue Arzneimittelformulierung enthaltend einen LTB₄ Antagonisten, welcher eine Benzamidingruppe aufweist, Verfahren zu deren Herstellung sowie deren Verwendung als Arzneimittel.

### Hintergrund der Erfindung

LTB₄ Antagonisten, welche eine Benzamidingruppe aufweisen, sind Verbindungen mit pharmakologisch wertvollen Eigenschaften. LTB₄ Antagonisten können beispielsweise bei der Behandlung von Arthritis, Asthma, chronisch obstruktiven Lungenerkrankungen, Psoriasis, Colitis ulcerosa, Alzheimer Krankheit, Schock, Reperfusions Schäden /Ischaemie, Cystischer Fibrose, Atherosklerose und Multipler Sklerose einen hohen therapeutischen Nutzen entfalten.

Solche Verbindungen sind bekannt z.B. aus den Internationalen Patentanmeldungen WO 93/16036, WO 94/11341, WO 96/02497, WO 97/21670, WO 98/11062, WO 98/11119, WO 01/25186 und PCT/EP01/00262.

Diese Verbindungen weisen die chemische Struktur der Formel I auf: 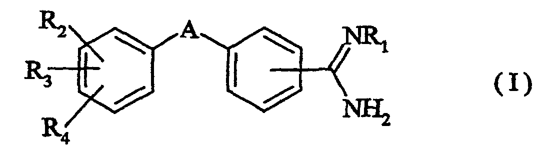 wobei
- A: eine Gruppe der Formel

-O-CₘH₂ₘ-O-(PHE)ₙ- (II)

bedeutet, worin
m eine ganze Zahl von 2 bis 6, vorzugsweise 2 bis 5 ist,
n 0 oder 1 ist,
PHE für eine gegebenenfalls durch ein oder zwei C₁-C₆ Alkylgruppen substituierte 1,4-Phenylengruppe, vorzugsweise eine durch in *ortho* -Position zum Sauerstoff verknüpfte C₂-C₄ Alkylgruppe substituierte 1,4-Phenylengruppe steht;
oder
- A: eine Gruppe der Formel 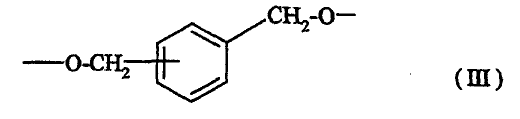 vorzugsweise der Formel 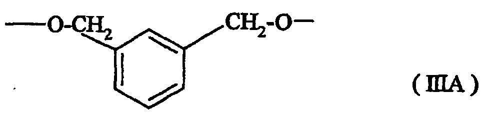 bedeutet,
- R₁: H, OH, CN, COR₁₀, oder CHO, vorzugsweise H oder COOR₁₀ bedeutet;
- R₂: H, Br, Cl, F, CF₃, CHF₂, OH, HSO₃-O-, C₁-C₆-Alkyl, C₁-C₆-Alkoxy, C₅-C₇-Cycloalkyl, CONR₈R₉, Aryl, O-Aryl, CH₂-Aryl, CR₅R₆-Aryl, oder C(CH₃)₂-R₇, vorzugsweise OH, HSO₃-O-, CONR₈R₉ oder CR₅R₆-Aryl bedeutet,
- R₃: H, C₁-C₆-Alkyl, C₁-C₆-Alkoxy, OH, Cl oder F, vorzugsweise H oder C₁-C₃-Alkoxy bedeutet,
- R₄: H oder C₁-C₆-Alkyl, vorzugsweise H bedeutet;
- R₅: C₁-C₄-Alkyl, CF₃, CH₂OH, COOH oder COO(C₁-C₄-Alkyl), vorzugsweise C₁-C₄-Alkyl, insbesondere Methyl bedeutet;
- R₆: H, C₁-C₄-Alkyl oder CF₃, vorzugsweise C₁-C₄-Alkyl, insbesondere Methyl bedeutet;
- R₇: CH₂OH, COOH, COO(C₁-C₄-Alkyl), CONR₈R₉ oder CH₂NR₈R₉ bedeutet;
- R₈: H, C₁-C₆-Alkyl, Phenyl, Phenyl-(C₁-C₆-alkyl), COR₁₀, COOR₁₀, CHO, CONH₂, CONHR₁₀, SO₂-(C₁-C₆-alkyl), SO₂-Phenyl, wobei die Phenyl Gruppe ein- oder zweifach durch Cl, F, CF₃, C₁-C₄-Alkyl, OH und/oder C₁-C₄-Alkoxy substituiert sein kann, vorzugsweise C₁-C₄-Alkyl, insbesondere Isopropyl bedeutet;
- R₉: H oder C₁-C₆-Alkyl, vorzugsweise H oder C₁-C₄-Alkyl, insbesondere Isopropyl bedeutet; oder
- R₈ und R₉: zusammengenommen eine C₄-C₆-Alkylene Gruppe bedeuten;
- R₁₀: C₁-C₆-Alkyl, C₅-C₇-Cycloalkyl, Aryl, Heteroaryl, Aralkyl oder Heteroaryl-(C₁-C₆-alkyl), vorzugsweise C₁-C₄-Alkyl bedeutet
wobei die in den Resten R₂ und R₁₀ aufgeführten Arylgruppen für Phenyl oder Naphthyl stehen, die Heteroaryl Gruppen für Pyrrol, Pyrazol, Imidazol, Furanyl, Thienyl, Pyridin oder Pyrimidin stehen und jeweils ein- oder mehrfach durch Cl, F, CF₃, C₁-C₄-Alkyl, OH, HSO₃-O- oder C₁-C₄-Alkoxy, vorzugsweise durch OH oder HSO₃-O- substituiert sein können.

Es ist Aufgabe der vorliegenden Erfindung, eine oral applizierbare pharmazeutische Formulierung bereitzustellen, die einen Wirkstoff der Formel I relativ rasch und vollständig freisetzt und somit zu einer erhöhten Bioverfügbarkeit dieses Wirkstoffs führt. Es ist ferner Aufgabe der vorliegenden Erfindung, eine Formulierung bereitzustellen, die durch eine gute Handhabbarkeit während des Herstellprozesses gekennzeichnet ist und dadurch die technische Herstellung in reproduzierbarer Art und Weise bei gleichbleibend hoher Qualität erlaubt.

### Detaillierte Beschreibung der Erfindung

Vorstehend genannte Aufgaben werden durch die im Folgenden detailliert beschriebene Formulierung gelöst.

Die vorliegende Erfindung betrifft eine Verbindung der Formel I enthaltende Tablette, welche eine Verbindung der Formel I, deren pharmakologisch akzeptablen Säureadditionssalze und Glucuronide, und mindestens einen pharmakologisch akzeptablen Hilfsstoff enthält, wobei die Tablette mindestens ein Netzmittel enthält.

Ein weiterer Gegenstand ist die Verwendung einer Tablette nach einem der Ansprüche 1 bis 14 zur Herstellung eines Arzneimittels mit erhöhter Bioverfügbarkeit zur Behandlung oder Vorbeugung von Krankheiten, bei denen LTB₄ Antagonisten therapeutisch oder präventiv eingesetzt werden können.

Der Wirkstoff der Formel I kann in der erfindungsgemäßen Formulierung in Form eines physiologisch verträglichen Säureadditionssalzes vorliegen. Als physiologisch verträgliche Säureadditionssalze werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind. Gegebenenfalls können zur Herstellung der Salze auch Mischungen der vorgenannten Säuren eingesetzt werden. Erfindungsgemäß bevorzugt sind die Salze der Formel I ausgewählt aus der Gruppe bestehend aus Hydrochlorid, Hydrobromid, Sulfat, Phosphat, Fumarat und Methansulfonat. Besonders bevorzugt sind die Salze ausgewählt aus Hydrochlorid, Hydrobromid und Fumarat. Der Wirkstoff kann gegebenenfalls in Form eines Hydrats vorliegen. Erfindungsgemäß bevorzugt wird die Verbindung der Formel I allerdings in Form der freien Base und in der wasserfreien Form der Tablette zugesetzt.

Besonders bevorzugte Verbindungen der Formel I sind die der Formeln IA, IB und IC, insbesondere IA:

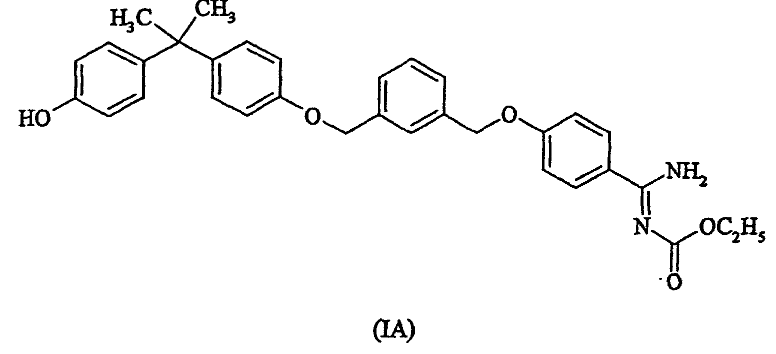

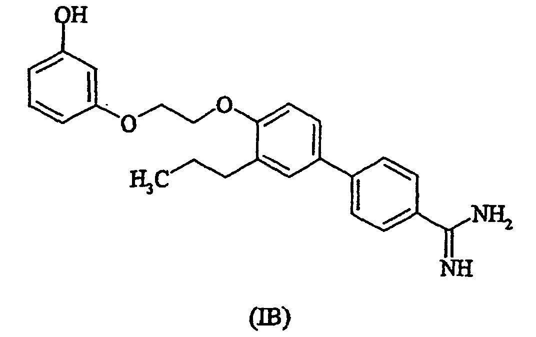

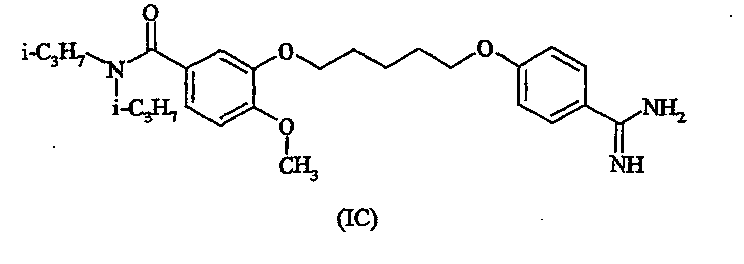

Die Verbindungen der Formel I, worin R₁ von Wasserstoff verschieden ist, sind in der Regel Prodrugs, die *in-vivo* in die entsprechenden Verbindungen der Formel I, worin R₁ Wasserstoff ist, umgewandelt werden.

Aus der Verbindung IA, entsteht beispielsweise *in-vivo* die Verbindung der Formel IA1: 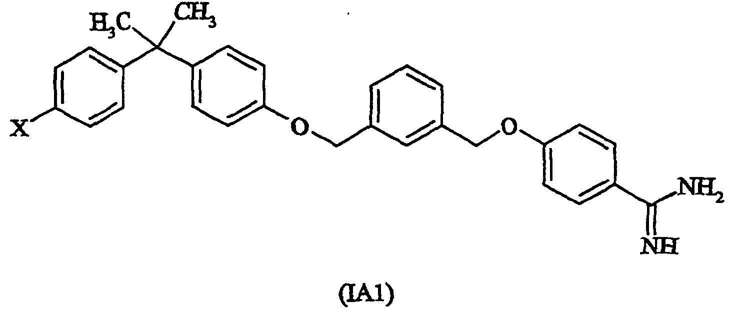 worin X OH, HSO₃-O- oder einen Kohlenhydratrest der Formel C₆H₁₁O₅-O- bedeutet.

Vorzugsweise wird der jeweilige Wirkstoff in kristalliner, unvermahlener Form oder gemahlener Form, insbesondere in strahlgemahlener Form eingesetzt, bei dem die Teilchengrößenverteilung innerhalb folgender Grenzen liegt: D₁₀ ≤ 3 µm, D₅₀ 3 bis 8 µm, D₉₀ ≤ 8 bis 30 µm. Bei den vorstehend genannten Zahlenangaben für D₁₀, D₅₀ und D₉₀ in µm (Mikrometer) handelt es sich um die Teilchengrößenbereiche innerhalb derer eine Durchgangssumme, von 10 Volumen-%, 50 Volumen-% bzw. 90 Volumen-% der gemessenen Partikel (kumulative Volumenverteilung) erreicht wird. Diese Werte wurden mit dem Verfahren der Laserdiffraktometrie bestimmt, im vorliegenden Fall speziell unter Anwendung einer sogenannten Trockendispergierung bei 2 bar Dispersionsdruck sowie einer Brennweite f = 500 mm, z.B. mit Hilfe eines Sympatec/RODOS Gerätes. Diese Methodik ist im Stand der Technik bekannt.

Wird im Rahmen der vorliegenden Erfindung auf Salze der Verbindungen der Formel I Bezug genommen, wird dies durch die Bezeichnung **I'** kenntlich gemacht. Ein explizite Bezugnahme auf die freie Base der Formel I erfolgt dagegen durch Verwendung der Bezeichnung **I**.

Bezogen auf die Gesamtmasse der erfindungsgemäßen Tabletten ist die Verbindung der Formel I, insbesondere IA erfindungsgemäß zu 0,2 bis 80 Gew-%, bevorzugt 0,7 bis 40 Gew-%, besonders bevorzugt etwa 5 bis 38 Gew-% enthalten. Besonders bevorzugt liegt der Anteil der freien Base von **I** zwischen 6 und 36 Gew-% bezogen auf die Gesamtmasse der Tablette.

Der Begriff "Netzmittel" wie er vor und nachstehend benutzt wird steht für einen Hilfsstoff, welcher die Oberflächenspannung von Wasser oder anderer Flüssigkeiten herabsetzt, so dass diese in die Oberflächen der erfindungsgemäßen Tablette eindringen und sie unter Verdrängung der Luft durchtränken und benetzen können. Die als Netzmittel eingesetzten Stoffe sind in der Regel grenzflächenaktive Tenside. Solche Tenside sind amphiphile (bifunktionelle) Verbindungen mit mindestens einem hydrophoben u. einem hydrophilen Molekülteil. Der hydrophobe Rest ist zumeist eine - möglichst lineare - Kohlenwasserstoff Kette mit acht bis 22 Kohlenstoff-Atomen. Spezielle Tenside können auch (Dimethyl)-Siloxan-Ketten oder perfluorierte Kohlenwasserstoff-Ketten als hydrophoben Molekülteil haben. Der hydrophile Rest ist entweder eine negativ oder positiv geladene (hydratisierbare) oder eine neutrale polare Kopfgruppe. Unter den Tensiden sind anionische Tenside, insbesondere die langkettige Alkylsulfate, insbesondere Natrium Laurylsulfat und Alkylbenzolsulfonate bevorzugt.

Erfindungsgemäß liegt das Gewichtsverhältnis zwischen den in der Tablette enthaltenen Komponente Netzmittel zu Wirkstoff **I** in einem Bereich von etwa 1:200 bis etwa 1:5, vorzugsweise zwischen etwa 1:150 und etwa 1:10.

Die erfindungsgemäße Arzneimittelformulierung enthält neben dem Wirkstoff ferner wenigstens einen Hilfsstoff als Füllstoff/Trockenbindemittel.

Im Rahmen der vorliegenden Erfindung kommt dabei Kohlenhydraten wie Laktose oder Mannose, insbesondere feinverteilte Laktose oder Zuckeralkoholen wie Mannitol, Sorbit oder Xylit, insbesondere Mannitol als Hilfsstoff eine besondere Bedeutung zu. Diese Hilfsstoffe haben sich bei der erfindungsgemäßen Formulierung als besonders vorteilhaft erwiesen. Ein bevorzugter Aspekt der vorliegenden Erfindung betrifft daher eine mindestens eine Verbindung der Formel I enthaltende Tablette, die neben dem Wirkstoffund dem Netzmittel Laktose, insbesondere feinverteilte Laktose, besonders bevorzugt Laktosemonohydrat oder Mannitol als Hilfsstoff enthält.

Erfindungsgemäß liegt das Gewichtsverhältnis zwischen den in der Tablette enthaltenen Komponenten Laktose oder Mannitol zu Wirkstoff **I** in einem Bereich von etwa 20:1 bis etwa 1:4. Vorzugsweise liegt das Verhältnis von Laktose zu **I** in einem Bereich von etwa 1,8:1 bis etwa 1:1,2, besonders bevorzugt in einem Bereich von etwa 1,4:1 bis 1,6:1. Bevorzugt liegt der Gewichtsanteil der Laktose bezogen auf die Gesamtmasse der erfindungsgemäßen Tablette in einem Bereich von etwa 1 - 70 Gew-%, bevorzugt zwischen etwa 2 - 60 Gew-%.

Vorzugsweise liegt das Verhältnis von Mannitol zu **I** in einem Bereich von etwa 20:1 bis etwa 1:2, besonders bevorzugt in einem Bereich von etwa 15:1 bis 3:1. Bevorzugt liegt der Gewichtsanteil von Mannitol bezogen auf die Gesamtmasse der erfindungsgemäßen Tablette in einem Bereich von etwa 20 - 90 Gew-%, bevorzugt zwischen etwa 40 - 75 Gew-%.

Die erfindungsgemäße Tablette kann neben Laktose, Netzmittel und Wirkstoff ferner weitere Hilfsstoffe bzw. Füllstoffe enthalten. Erfindungsgemäß bevorzugt können solche Verbindungen eingesetzt werden, die als Bindemittel fungieren können.

Der Begriff "Bindemittel" steht vor- und nachstehend für solche Hilfsmittel, die geeignet sind, andere Komponenten miteinander abzubinden. Erfindungsgemäß bevorzugte Bindemittel sind ausgewählt aus der Gruppe bestehend aus:
Pulvercellulose, mikrokristalline Cellulose, Sorbitol, Stärke, Polyvinylpyrrolidon (Povidon), Copolymerisaten von Vinylpyrrolidon mit anderen Vinylderivaten (Copovidone), Cellulosederivaten, insbesondere Methylhydroxypropylcellulose, z.B. Methocel A 15 LV, und Gemische dieser Verbindungen. Bevorzugt sind als Bindemittel Pulvercellulose, besonders mikrokristalline Cellulose und/oder Copovidone enthalten. Am meisten bevorzugt ist ein Gemisch aus mikrokristalliner Cellulose und einem Co-polymerisat aus Vinylpyrrolidon und Vinylacetat, nämlich Copovidone VA 64, wobei das Verhältnis von Vinylpyrrolidon und Vinylacetat etwa 3 :2 (m/m) ist. In der Regel weist die erfindungsgemäße Tablette ein Gewichtsverhältnis von mikrokristalliner Cellulose zu Copovidone VA 64 von 20:1 bis 1:1, vorzugsweise 15:1 bis 2:1, insbesondere etwa 10:1 bis 3:1 auf. Aufgrund dieser besonders bevorzugten Bindemittel-Kombination aus mikrokristalliner Cellulose und Copovidone werden Tabletten mit erhöhter Bioverfügbarkeit der Verbindungen der Formel I erhalten.

Wird eines der vorstehend genannten Bindemittel der erfindungsgemäßen Formulierung zugesetzt, so beträgt das Gewichtsverhältnis von Laktose zu Bindemittel bevorzugt etwa 10:1 bis etwa 1:2, bevorzugt etwa 5:1 bis etwa 1:1, besonders bevorzugt etwa 4,5:1 bis 3,5:2.

Wird eines der vorstehend genannten Bindemittel der erfindungsgemäßen Formulierung zugesetzt, so beträgt das Gewichtsverhältnis von Mannitol zu Bindemittel bevorzugt etwa 10:1 bis etwa 1:2, bevorzugt etwa 5:1 bis etwa 1:1, besonders bevorzugt etwa 3,5:1 bis 3,0:1.

Die erfindungsgemäße Tablette kann neben vorstehend genannten Bestandteilen ferner Zerfallsmittel enthalten. Im Rahmen der vorliegenden Erfindung können diese Zerfallsmittel gegebenenfalls auch als Sprengmittel bezeichnet werden. Diese sind erfindungsgemäß bevorzugt ausgewählt aus der Gruppe bestehend aus Natriumstärkeglykolat, kreuzvernetztes Polyvinylpyrrolidone (Crospovidon), Croscarmellose Natriumsalz (Cellulose carboxymethylether Natriumsalz, quervernetzt), Natrium-carboxymethylcellulose (?), getrocknete Maisstärke und Gemische davon. Besonders bevorzugt gelangen im Rahmen der vorliegenden Erfindung Natriumstärkeglykolat, Crospovidon und, bevorzugt Crospovidon oder Croscarmellose Natriumsalz zur Anwendung. Werden vorstehend genannte Zerfallsmittel verwendet, liegt ihr Gewichtsanteil bezogen auf die Gesamtmasse der erfindungsgemäßen Tablette bevorzugt in einem Bereich von etwa 0,5 - 10 Gew-%, besonders bevorzugt bei etwa 1,5 - 7,5 Gew-%.

Die erfindungsgemäße Tablette kann als weitere Bestandteile ferner Fließ- bzw. Fließregulierungsmittel sowie Schmiermittel enthalten. Hierzu kommen im Rahmen der vorliegenden Erfindung beispielsweise in Betracht Siliciumdioxid, Talkum, Stearinsäure, Natrium-Stearylfumarat, Magnesiumstearat und Glyceroltribehenat. Erfindungsgemäß bevorzugt wird Magnesiumstearat verwendet. Werden vorstehend genannte Fließ- bzw. Fließregulierungsmittel bzw. Schmiermittel verwendet, liegt ihr Gewichtsanteil bezogen auf die Gesamtmasse der erfindungsgemäßen Tablette bevorzugt in einem Bereich von etwa 0,1 - 10 Gew-%, bevorzugt bei etwa 0,5 - 5 Gew-%, besonders bevorzugt zwischen 0,6 und 1,5 Gew-%.

Weiterhin kann die erfindungsgemäße Tablette einen oder mehrere synthetische oder natürliche, pharmazeutisch verträgliche Farbstoffe enthalten, vorzugsweise Indigo carmine. Werden vorstehend genannte Farbstoffe verwendet liegt ihr Gewichtsanteil bezogen auf die Gesamtmasse der erfindungsgemäßen Tablette bei 0,01 bis 0,5 Gew-%.

Besonders bevorzugt ist eine Tablette, welche im wesentlichen aus folgenden Bestandteilen besteht:
● einer Verbindung der Formel I, vorzugsweise 5 Gew.-% bis 50 Gew.-%, insbesondere 6 Gew.- % bis 45 Gew.-% einer Verbindung der Formel IA;
● mikrokristalliner Cellulose, vorzugsweise 0,5 Gew.-% bis 25 Gew.-%, insbesondere 5 Gew.-% bis 20 Gew.-% mikrokristalliner Cellulose;
● Laktose, vorzugsweise 15 Gew.-% bis 80 Gew.-%, insbesondere 20 Gew.-% bis 75 Gew.-% Laktose; oder
   Mannitol, vorzugsweise 20 Gew.-% bis 90 Gew.-%, insbesondere 40 Gew.-% bis 75 Gew.-% Mannitol;
● gegebenenfalls einem Co-polymerisat aus Vinylpyrrolidon und Vinylacetat, vorzugsweise 0,2 Gew.-% bis 5 Gew.-%, insbesondere 0,5 Gew.-% bis 2,5 Gew.-% Copovidone V64,
● Natriumlaurylsulfat, vorzugsweise 0,01 Gew.-% bis 10 Gew.-%, insbesondere 0,06 Gew.-% bis 7 Gew.-% Natriumlaurylsulfat;
● kreuzvernetztem Polyvinylpyrrolidon, vorzugsweise 0,4 Gew.-% bis 8 Gew.-%, insbesondere 0,8 Gew.-% bis 6 Gew.-%; oder quervernetztes Cellulose Carboxymethylether Natriumsalz, insbesondere Croscarmellose Natriumsalz;
● Magnesiumstearat, vorzugsweise 0,1 Gew.-% bis 5 Gew.-%, insbesondere 0,5 Gew.-% bis 1,5 Gew.-% Magnesiumstearat; und
● gegebenenfalls einem Farbstoff.

Die erfindungsgemäße Tablette kann durch direktes Mischen und Verpressen der Bestandteile oder durch Granulieren und Verpressen hergestellt werden. Zur Herstellung der erfindungsgemäßen Tablette kann beispielsweise wie nachfolgend beschrieben vorgegangen werden.

Der Wirkstoff der Formel I, das Netzmittel und gegebenenfalls ein Co-polymerisat von Vinylpyrrolidon und Vinylacetat als Bindemittel werden in Wasser gelöst bzw. suspendiert, mit den Hilfsstoffen, insbesondere Laktose Monohydrat und gegebenenfalls mikrokristalliner Cellulose als weiterem Bindemittel granuliert, gesiebt und anschließend getrocknet. Das erhaltene Produkt wird gegebenenfalls mit dem Fließmittel, insbesondere Magnesiumstearat gemischt und gesiebt. Anschließend wird gegebenenfalls ein Sprengmittel, insbesondere Crospovidone zugesetzt. Das so erhaltene Wirkstoff-Hilfstoffgemisch wird anschließend auf einer geeigneten Tablettenpresse zu den erfindungsgemäßen Tabletten verpresst.

Die Presskräfte, die benötigt werden, Tabletten der geeigneten Bruchfestigkeiten und damit der gewünschten Zerfallszeiten herzustellen, sind von den Formen und Größen der verwendeten Stempelwerkzeugen abhängig. Vorzugsweise ist Presskraft in einem Bereich von 2 - 20 kN,. Höhere Presskräfte können zu Tabletten mit verlangsamter WirkstoffFreisetzung fuhren. Niedrigere Presskräfte können zu mechanisch instabilen Tabletten fuhren. Die Tablettenkerne können unterschiedliche Formate aufweisen, bevorzugt sind runde biplane oder bikonvexe und ovale oder oblong Formen.

### Beispiel 1:

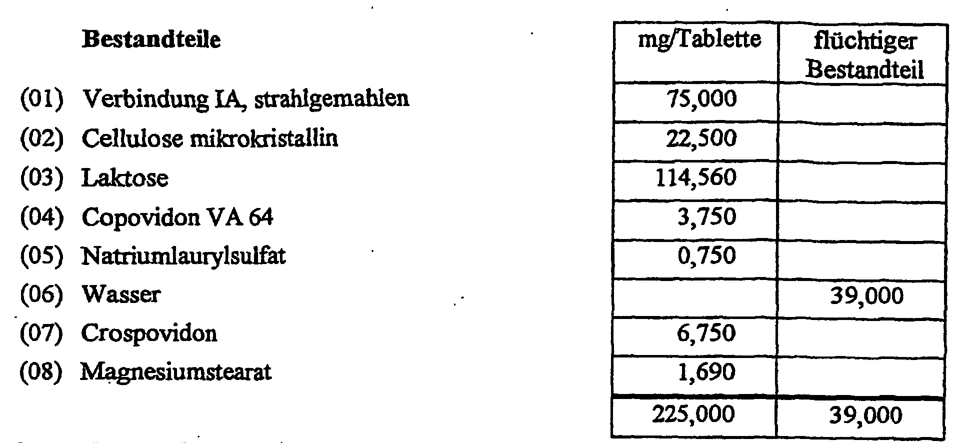

### Herstellung (Variante 1)

### 1 Ansatz = 2992,5 g = 13.300 Tabletten

### 1. Vormischung

In einem geeigneten Intensivmischer, z.B. Diosna P10, werden 997,500 g strahlgemahlene Verbindung der Formel IA, 299,250 g mikrokristalline Cellulose und 1523,648 g Laktose Monohydrat 3 Minuten gemischt.

### 2. Granulierflüssigkeit

In einem geeigneten Gefäß werden 518,700 g Wasser vorgelegt. Mit einem geeigneten Rührer werden 49,875 g Copovidon VA 64 langsam eingerührt. Nachdem das Polividon VA 64 sich gelöst hat, werden 9,975 g Natriumlaurylsulfat eingerührt.

### 3. Granulat

Die Vormischung 1. wird nach Zugabe der Granulierflüssigkeit 2. mit Hilfe eines Flüssigkeitstrichters feucht gemischt und in einem Intensivmischer granuliert.

### Feuchtsieben

Das so hergestellte feuchte Granulat wird mit einer geeigneten Siebmaschine, z.B Erweka AR401 bei einer Maschenweite von 1,6 mm feuchtgesiebt.

### Trocknen

In einem geeigneten Trockenschrank, z.B. einem WTB Binder, wird das Granulat bei einer Schichtdicke von etwa 1 cm bei einer Temperatur von 50 °C 10 Stunden getrocknet.

### Trockensieben

Mit einer geeigneten Siebmaschine, z.B Erweka AR401 wird das getrocknete Granulat bei einer Maschenweite von 0,8 mm trockengesiebt.

### 4. Endmischung

In einem geeigneten Gefäß mit einem geeigneten Mischer, z.B. Röhnradmischer Rm 100 werden 2880,248 g des gesiebten Granulats 3. bei 30 Upm 89,775 g Crospovidon zugeben und 10 Minuten homogen gemischt. Anschließend werden der Mischung 22,477 g Magnesiumstearat zugeben und 5 Minuten homogen gemischt.

### 5. Tablettierung

Auf einer geeigneten Tablettenpresse, z.B. EK 0 (Presswerkzeug: 9 mm mit Wölbungsradius 13,5 mm und Facette), werden bei einer Pressgeschwindigkeit von 25 St./Min. 2992,500 g der Endmischung 4. zu Tabletten von jeweils 225 mg verpresst.

Die so erhaltenen Tabletten weisen folgende Eigenschaften auf:

| | |
|---|---|
| Gewicht | 225 mg |
| Durchmesser | 9 mm |
| Höhe | etwa 3,6 mm |
| Bruchfestigkeit | 100 N (n = 10 St) |
| Zerfall | ≤ 8 Minuten |
| Friabilität - Abrieb | < 0,5 % |

### Beispiel 2:

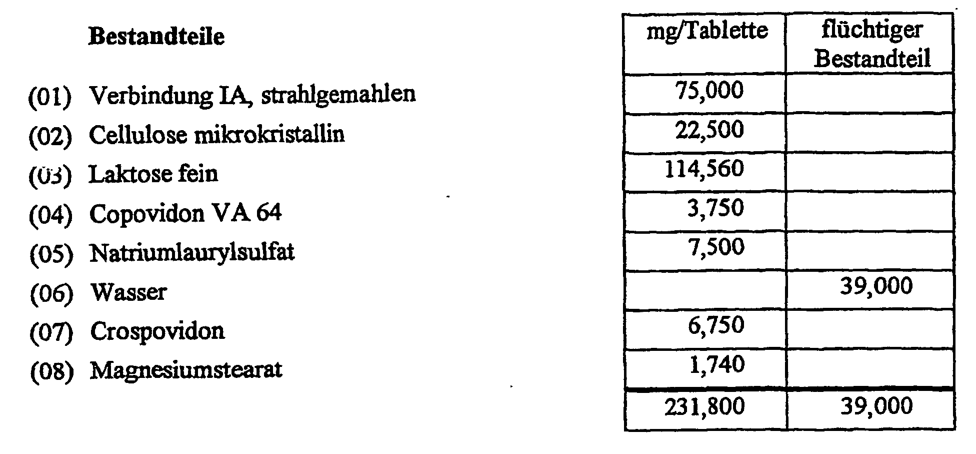

Die Herstellung erfolgt analog Beispiel 1. Die erhaltenen Tabletten weisen vergleichbare physikalische Daten auf.

### Beispiel 3:

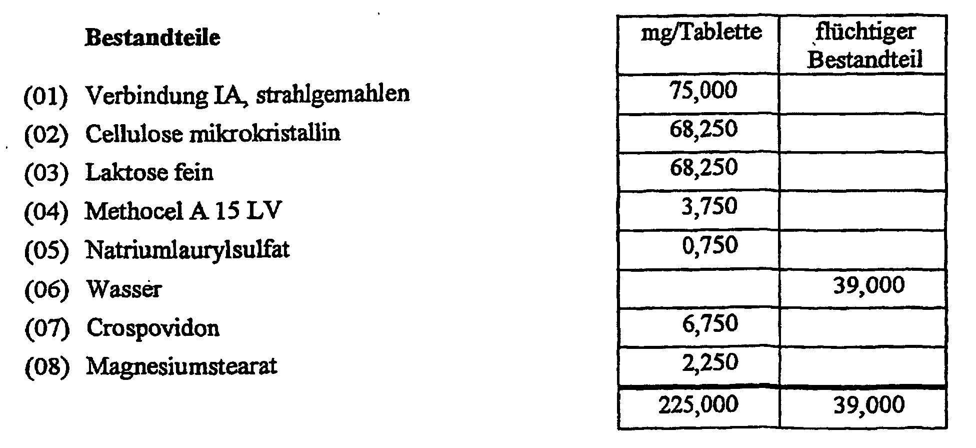

Die Herstellung erfolgt analog Beispiel 1. Die erhaltenen Tabletten weisen vergleichbare physikalische Daten auf, aber eine niedrigere Härte und eine langsamere Auflösung auf.

### Beispiel 4:

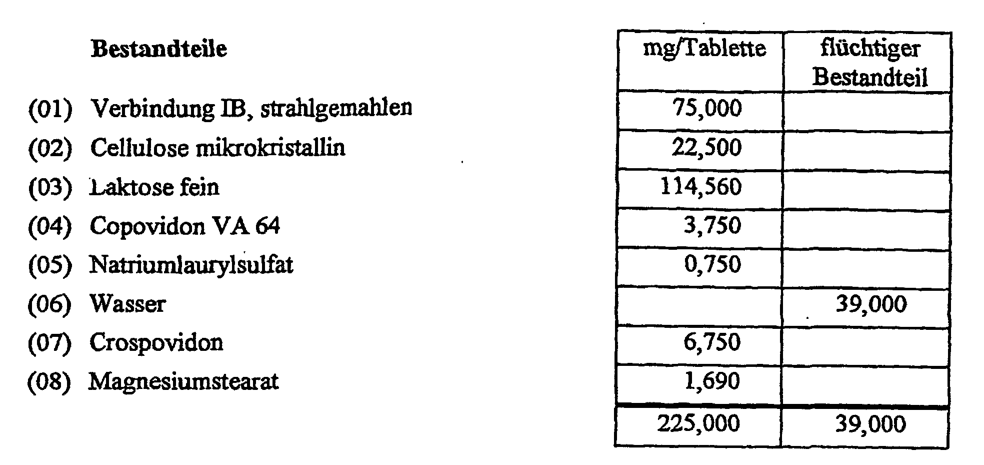

Die Herstellung erfolgt analog Beispiel 1. Die erhaltenen Tabletten weisen vergleichbare physikalische Daten auf

### Beispiel 5:

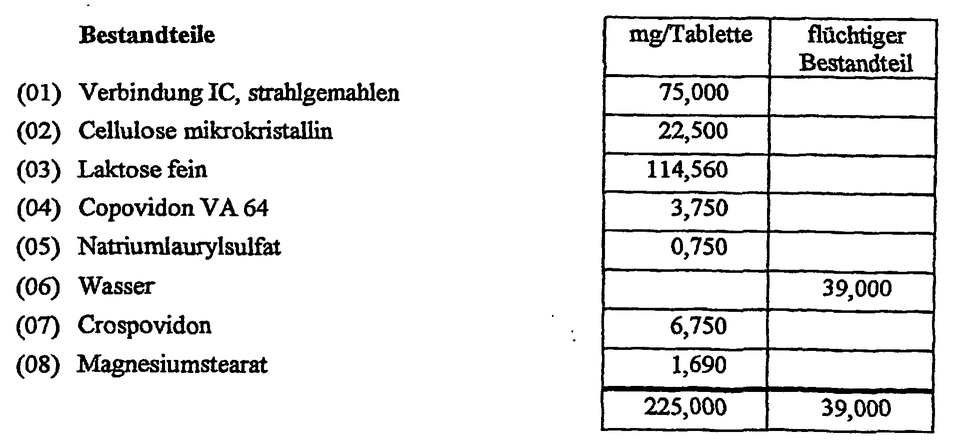

Die Herstellung erfolgt analog Beispiel 1. Die erhaltenen Tabletten weisen vergleichbare physikalische Daten auf.

### Beispiel 6

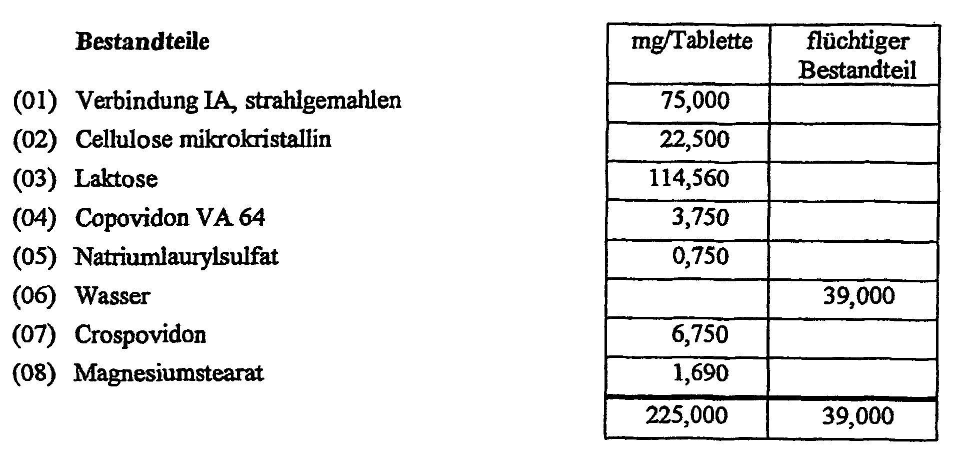

### Herstellung (Variante 2)

### 1. Vormischung

In einem geeigneten Eintopfgranulierer, z.B. Zanchetta Roto P 50, werden 4,6500 kg strahlgemahlene Verbindung der Formel IA, 1,3950 kg mikrokristalline Cellulose, 7,1028 kg Laktose und 0,4185 kg Crospovidon 3 Minuten gemischt.

### 2. Granulierflüssigkeit

In einem geeigneten Gefäß werden 2,1762 kg Wasser (entspricht 90% der Menge von Verfahren 1) vorgelegt. Mit einem geeigneten Rührer werden 0,2325 kg Copovidon VA 64 langsam eingerührt. Nachdem das Polividon VA 64 sich gelöst hat, werden 0,0465 kg Natriumlaurylsulfat eingerührt.

### 3. Granulat

Die Vormischung 1. wird nach Zugabe der Granulierflüssigkeit feucht gemischt und in einem Eintopfgranulierer granuliert.

### Trocknen

Das Granulat wird in einem Eintopfgranulierer bei einer Temperatur von 49°C 50 Minuten getrocknet.

### Trockensieben

Mit einer geeigneten Siebmaschine, z.B. Comil 197 S, wird das getrocknete Granulat bei einer Siebgröße von RS 2007 trockengesiebt.

### 4. Endmischung

In einem geeigneten Freifallmischer, z.B. Servolift Kubus 60 l, werden 13,8453 kg des gesiebten Granulats 3. und 0,1047 kg Magnesiumstearat bei 10 Upm 5 Minuten homogen gemischt.

### 5. Tablettierung

Auf einer geeigneten Tablettenpresse, z.B. Fette P 1200 (Presswerkzeug: 9 mm mit Wölbungsradius 13,5 mm und Facette), werden bei einerPressgeschwindigkeit von 100.000 St./h 13,9500 kg der Endmischung 4. zu Tabletten von jeweils 225 mg verpresst.

### Beispiel 7

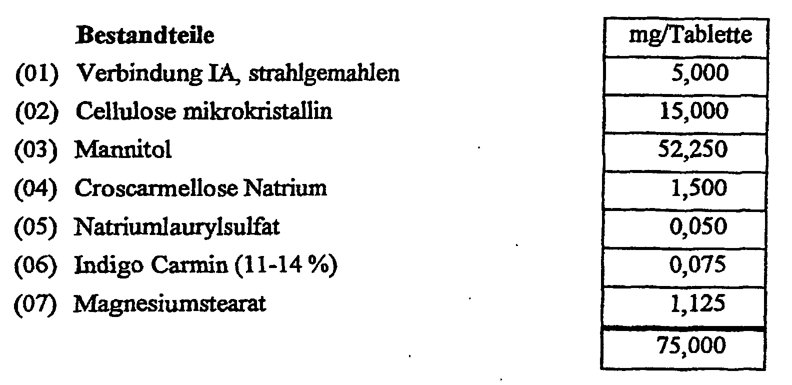

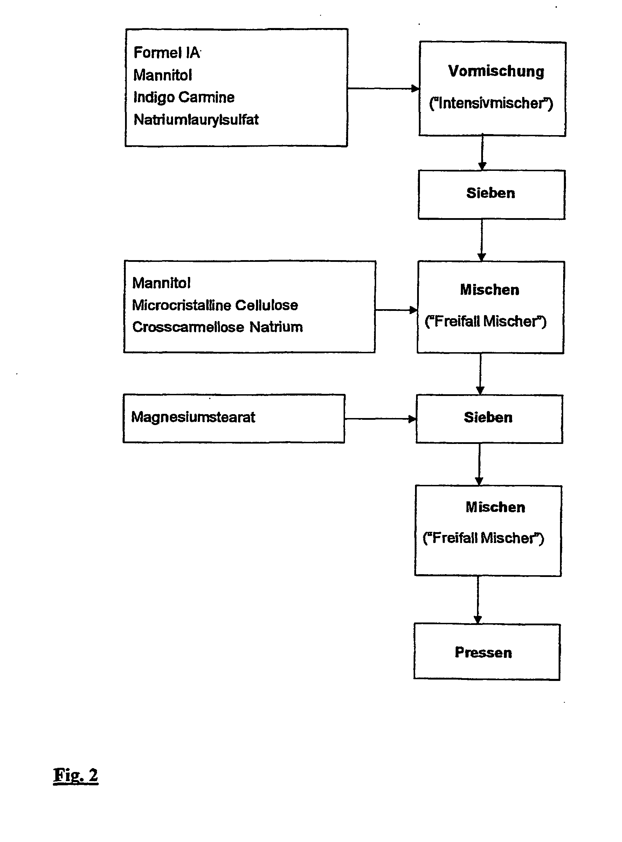

Die direkte Verpressung umfasst die Herstellung einer Vormischung der Bestandteile (01), (05), (06) und eines Teiles von (03) mit einem Diosna® Intensivmischer. Die Vormischung wird gesiebt und mit den Bestandteilen (02), (04) und dem restlichen (03) in einem Turbula® Freifallmischer gemischt. Nach erneutem Sieben wird der Bestandteil (07) hinzugefügt. Ein entsprechendes Ablaufschema ist in Figure 2 zu sehen.

Bikonvexe, 6 mm dicke Tabletten mit abgerundeten Kanten enthaltend 5 mg der Verbindung der Formel IA bei einem Gesamtgewicht von 75 mg werden gepresst

### Beispiel 8

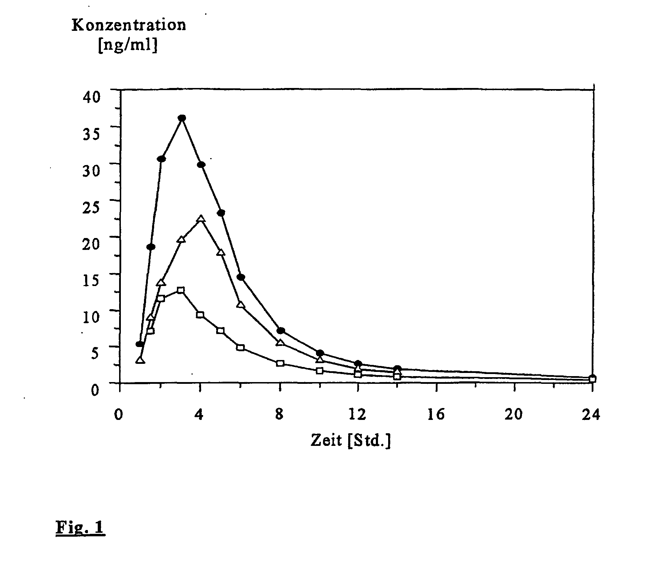

Die erfindungsgemäßen Tabletten der Beispiele 1 bis 3 werden gesunden, männlichen Probanden oral als Einmalgabe verabreicht. Die Plasmakonzentration des jeweiligen Wirkstoffes wird in zeitlichen Abständen bestimmt. Die so erhaltenen Mittelwerts-Daten sind in Figur 1 graphisch dargestellt. In Fig. 1 ist die Konzentration in ng/ml des aus der Verbindung der Formel IA *in-vivo* erzeugten Glykosids der Verbindung der Formel IA1 im Blutplasma gegen die Zeit in Stunden aufgetragen. Demnach erhält man die höchsten Wirkstoffkonzentrationen im Plasma mit der Tablette gemäß Beispiel 1.

Die in Fig. 1 verwendeten Symbole weisen folgende Bedeutungen auf
―●―: Tablette des Beispiels 1
―Δ―: Tablette des Beispiels 2
―□―: Tablette des Beispiels 3

## Patentansprüche

1. Arzneimittelformulierung in Form einer Tablette enthaltend einen LTB₄ Antagonisten der Formel I, 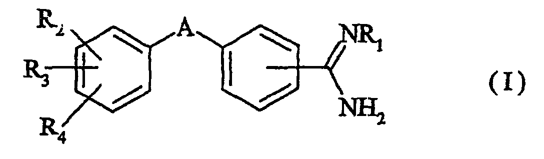 wobei
A eine Gruppe der Formel
-O-CₘH₂ₘ-O-(PHE)ₙ- (II)
bedeutet, worin
m eine ganze Zahl von 2 bis 6 ist,
n 0 oder 1 ist,
PHE für eine gegebenenfalls durch ein oder zwei C₁-C₆ Alkylgruppen substituierte 1,4-Phenylengruppe steht;
oder
A eine Gruppe der Formel 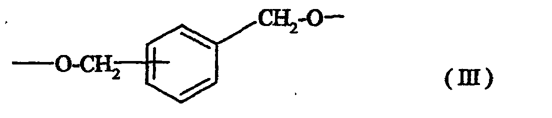 bedeutet,
R₁ H, OH, CN, COR₁₀, COOR₁₀ oder CHO bedeutet;
R₂ H, Br, Cl, F, CF₃, CHF₂, OH, HSO₃-O-, C₁-C₆-Alkyl, C₁-C₆-Alkoxy, C₅-C₇-Cycloalkyl, CONR₈R₉, Aryl, O-Aryl, CH₂-Aryl, CR₅R₆-Aryl, oder C(CH₃)₂-R₇ bedeutet,
R₃ H, C₁-C₆-Alkyl, C₁-C₆-Alkoxy, OH, Cl oder F bedeutet,
R₄ H oder C₁-C₆-Alkyl bedeutet;
R₅ C₁-C₄-Alkyl, CF₃, CH₂OH, COOH oder COO(C₁-C₄-Alkyl) bedeutet;
R₆ H, C₁-C₄-Alkyl oder CF₃ bedeutet;
R₇ CH₂OH, COOH, COO(C₁-C₄-Alkyl), CONR₈R₉ oder CH₂NR₈R₉ bedeutet;
R₈ H, C₁-C₆-Alkyl, Phenyl, Phenyl-(C₁-C₆-alkyl), COR₁₀, COOR₁₀, CHO, CONH₂, CONHR₁₀, SO₂-(C₁-C₆-alkyl), SO₂-Phenyl, wobei die Phenyl Gruppe ein- oder zweifach durch Cl, F, CF₃, C₁-C₄-Alkyl, OH und/oder C₁-C₄-Alkoxy substituiert sein kann, bedeutet;
R₉ H oder C₁-C₆-Alkyl bedeutet; oder
R₈ und R₉ zusammengenommen eine C₄-C₆-Alkylene Gruppe bedeuten;
R₁₀ C₁-C₆-Alkyl, C₅-C₇-Cycloalkyl, Aryl, Heteroaryl, Aralkyl or Heteroaryl-(C₁-C₆-alkyl) bedeutet
wobei die in den Resten R₂ und R₁₀ aufgeführten Arylgruppen für Phenyl oder Naphthyl stehen, die Heteroaryl Gruppen für Pyrrol, Pyrazol, Imidazol, Furanyl, Thienyl, Pyridin oder Pyrimidin stehen und jeweils ein- oder mehrfach durch Cl, F, CF₃, C₁-C₄-Alkyl, OH, HSO₃-O- oder C₁-C₄-Alkoxy substituiert sein können,
sowie deren pharmakologisch akzeptablen Säureadditionssalze und Glykoside und 0-Sulfate;
und mindestens einen pharmakologisch akzeptablen Hilfsstoff, **dadurch gekennzeichnet, dass** die Tablette mindestens ein Netzmittel enthält.

2. Tablette nach Anspruch 1, **dadurch gekennzeichnet, dass** der LTB₄ Antagonist eine Verbindung ausgewählt aus den Formeln IA, IB und IC ist: 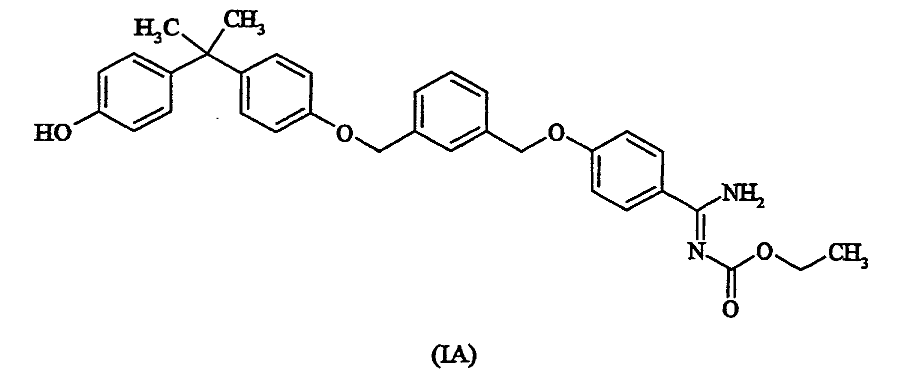 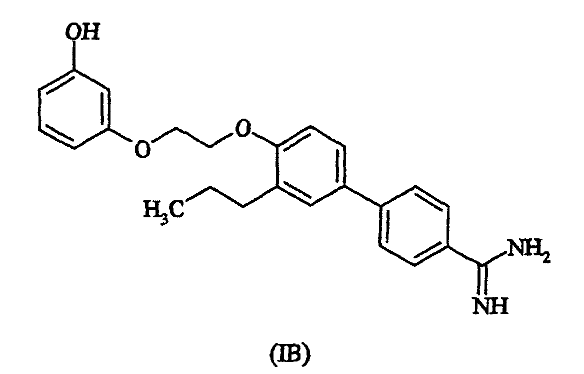 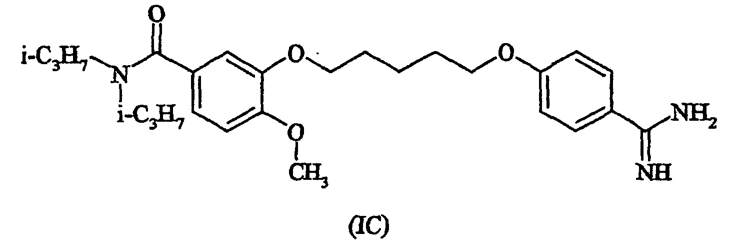 ist.

3. Tablette nach einem der Ansprüche 1 oder 2, **dadurch gekennzeichnet, dass** das Netzmittel ein anionischer grenzflächenaktiver Stoff ist.

4. Tablette nach einem der Ansprüche 1 bis 3, **dadurch gekennzeichnet, dass** das Netzmittel Natriumlaurylsulfat ist.

5. Tablette nach einem der Ansprüche 1 bis 4, **dadurch gekennzeichnet, dass** sie als Hilfsstoff Laktose oder Mannitol enthält.

6. Tablette nach einem der Ansprüche 1 bis 5, **dadurch gekennzeichnet, dass** sie zusätzlich mindestens ein Trockenbindemittel, mindestens ein Schmiermittel, mindestens ein Zerfallsmittel und/oder mindestens ein Trennmittel und gegebenenfalls einen Farbstoff enthält.

7. Tablette nach Anspruch 6, **dadurch gekennzeichnet, dass** das Trockenbindemittel ausgewählt ist aus der Gruppe bestehend aus Pulvercellulose, mikrokristalliner Cellulose, Stärke, Polyvinylpyrrolidon, Co-polymerisaten von Vinylpyrrolidon mit anderen Vinylderivaten, Cellulosederivaten und Gemischen dieser Verbindungen.

8. Tablette nach Anspruch 6 oder 7, **dadurch gekennzeichnet, dass** das Trockenbindemittel mikrokristalliner Cellulose oder ein Gemisch aus mikrokristalliner Cellulose und einem Co-polymerisat von Vinylpyrrolidon und Vinylacetat ist.

9. Tablette nach einem der Ansprüche 1 bis 8, **dadurch gekennzeichnet, dass** sie 0,2 bis 80 Gew.-%, bevorzugt 0,7 bis 40 Gew.-% einer Verbindung der Formel I enthält.

10. Tablette nach einem der Ansprüche 1 bis 9, **dadurch gekennzeichnet, dass** das Gewichtsverhältnis zwischen dem Netzmittel und dem Wirkstoff der Formel I in einem Bereich von etwa 1 : 200 bis etwa 1 : 5, vorzugsweise zwischen etwa 1 : 150 bis etwa 1 : 10 beträgt.

11. Tablette nach einem der Ansprüche 5 bis 10, **dadurch gekennzeichnet, dass** das Gewichtsverhältnis zwischen Laktose oder Mannitol und dem Wirkstoff der Formel I im Bereich von etwa 20 : 1 bis etwa 1 : 4, bevorzugt etwa 12 : 1 bis etwa 1 : 1,2 liegt.

12. Tablette nach einem der Ansprüche 5 bis 11, **dadurch gekennzeichnet, dass** das Gewichtsverhältnis zwischen Laktose und dem Wirkstoff der Formel I im Bereich von etwa 4 : 1 bis etwa 1 : 4, bevorzugt etwa 1,8 : 1 bis etwa 1 : 1,2 liegt.

13. Tablette nach einem der Ansprüche 5 bis 11, **dadurch gekennzeichnet, dass** das Gewichtsverhältnis zwischen Mannitol und dem Wirkstoff der Formel I in einem Bereich von etwa 20:1 bis etwa 1:2, besonders bevorzugt in einem Bereich von etwa 15:1 bis 3:1.

14. Tablette nach einem der Ansprüche 6 bis 13, **dadurch gekennzeichnet, dass** der Gewichtsanteil des Zerfallsmittels bezogen auf die Gesamtmasse der Tablette in einem Bereich von etwa 0,5 bis 10 Gew.-% liegt.

15. Tablette nach einem der Ansprüche 5 bis 14, **dadurch gekennzeichnet, dass** der Gewichtsanteil der Fließ-, Schmier- und Trennmittels bezogen auf die Gesamtmasse der Tablette in einem Bereich von etwa 0,1 bis 5 Gew.-% liegt.

16. Tablette nach einem der Ansprüche 1 bis 15, **dadurch gekennzeichnet, dass** sie im wesentlichen aus folgenden Bestandteilen besteht:
● einer Verbindung der Formel I;
● mikrokristalliner Cellulose;
● Laktose oder Mannitol;
● gegebenenfalls einem Co-polymerisat aus Vinylpyrrolidon und Vinylacetat;
● Natriumlaurylsulfat;
● kreuzvernetztem Polyvinylpyrrolidon oder ein quervernetztes Cellulosecarboxymethylether Natriumsalz, insbesondere Croscarmellose Natriumsalz;
● Magnesiumstearat; und
● gegebenenfalls einem pharmazeutisch akzeptablen Farbstoff.

17. Verfahren zur Herstellung einer Tablette nach einem der Ansprüche 1 bis 16 durch
(a) Mischen einer Verbindung der Formel I mit einem Netzmittel und gegebenenfalls weiteren pharmakologisch akzeptablen Hilfsstoffen gegebenenfalls in Gegenwart eines flüchtigen Verdünnungsmittels;
(b) Sieben des erhaltenen Gemischs;
(c) gegebenenfalls Zusatz eines Schmiermittels, und
(d) Pressen mit einer geeigneten Tablettenpresse.

18. Verwendung einer Tablette nach einem der Ansprüche 1 bis 16 zur Herstellung eines Arzneimittels zur Behandlung oder Vorbeugung von Krankheiten, bei denen LTB₄ Antagonisten therapeutisch oder präventiv eingesetzt werden können.

19. Verwendung einer Tablette nach einem der Ansprüche 1 bis 16 zur Herstellung eines Arzneimittels zur Behandlung oder Vorbeugung von Arthritis, Asthma, chronisch obstruktiven Lungenerkrankungen, Psoriasis, Colitis ulcerosa, Alzheimer Krankheit, Schock, Reperfusions Schäden /Ischaemie, Cystischer Fibrose, Atherosklerose und Multipler Sklerose.

## Claims

1. Pharmaceutical formulation in the form of a tablet containing an LTB₄ antagonist of formula I, 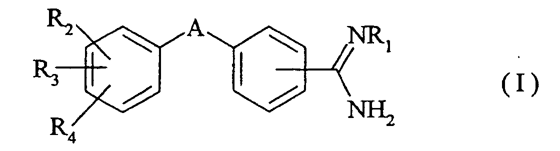 wherein
A denotes a group of formula
-O-CₘH₂ₘ-O-(PHE)ₙ- (II)
wherein
m is an integer from 2 to 6,
n is 0 or 1,
PHE denotes a 1,4-phenylene group optionally substituted by one or two C₁-C₆ alkyl groups;
or
A denotes a group of formula 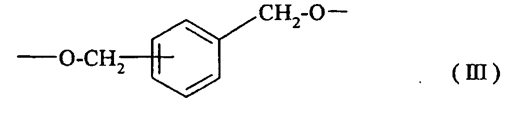
R₁ denotes H, OH, CN, COR₁₀ or CHO;
R₂ denotes H, Br, Cl, F, CF₃, CHF₂, OH, HSO₃-O, C₁-C₆-alkyl, C₁-C₆-alkoxy, C₅-C₇-cycloalkyl, CONR₈R₉, aryl, O-aryl, CH₂-aryl, CR₅R₆-aryl, or C(CH₃)₂-R₇,
R₃ denotes H, C₁-C₆-alkyl, C₁-C₆-alkoxy, OH, Cl or F,
R₄ denotes H or C₁-C₆-alkyl;
R₅ denotes C₁-C₄-alkyl, CF₃, CH₂OH, COOH or COO(C₁-C₄-alkyl);
R₆ denotes H, C₁-C₄-alkyl or CF₃;
R₇ denotes CH₂OH, COOH, COO(C₁-C₄-alkyl), CONR₈R₉ or CH₂NR₈R₉;
R₈ denotes H, C₁-C₆-alkyl, phenyl, phenyl-(C₁-C₆-alkyl), COR₁₀, COOR₁₀, CHO, CONH₂, CONHR₁₀, SO₂-(C₁-C₆-alkyl), SO₂-phenyl, while the phenyl group may be mono- or disubstituted by Cl, F, CF₃, C₁-C₄-alkyl, OH and/or C₁-C₄-alkoxy;
R₉ denotes H or C₁-C₆-alkyl; or
R₈ and R₉ taken together represent a C₄-C₆-alkylene group;
R₁₀ denotes C₁-C₆-alkyl, C₅-C₇-cycloalkyl, aryl, heteroaryl, aralkyl or heteroaryl-(C₁-C₆-alkyl),
while the aryl groups mentioned in groups R₂ and R₁₀ denote phenyl or naphthyl, the heteroaryl groups denote pyrrole, pyrazole, imidazole, furanyl, thienyl, pyridine or pyrimidine and may each be mono- or polysubstituted by Cl, F, CF₃, C₁-C₄-alkyl, OH, HSO₃-O or C₁-C₄-alkoxy,
as well as the pharmacologically acceptable acid addition salts and glycosides and O-sulphates thereof;
and at least one pharmacologically acceptable excipient, **characterised in that** the tablet contains at least one wetting agent.

2. Tablet according to claim 1, **characterised in that** the LTB₄ antagonist is a compound selected from among formulae IA, IB and IC:

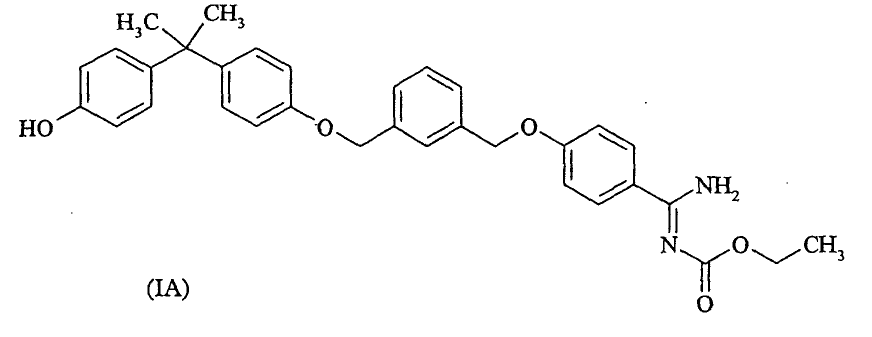

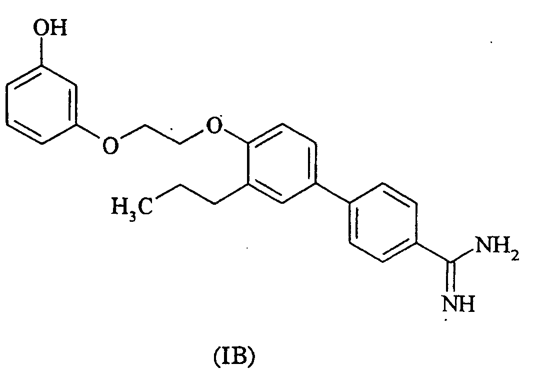

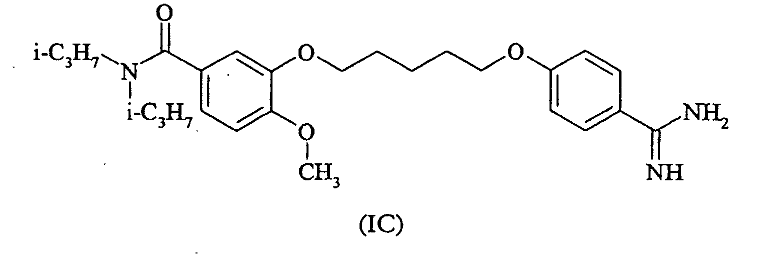

3. Tablet according to one of claims 1 or 2, **characterised in that** the wetting agent is an anionic surface-active substance.

4. Tablet according to one of claims 1 to 3, **characterised in that** the wetting agent is sodium laurylsulphate.

5. Tablet according to one of claims 1 to 4, **characterised in that** it contains lactose or mannitol as excipient.

6. Tablet according to one of claims 1 to 5, **characterised in that** it additionally contains at least one dry binder, at least one lubricant, at least one disintegrant and/or at least one separating agent and optionally a colouring.

7. Tablet according to claim 6, **characterised in that** the dry binder is selected from among powdered cellulose, microcrystalline cellulose, starch, polyvinylpyrrolidone, copolymers of vinylpyrrolidone with other vinyl derivatives, cellulose derivatives and mixtures of these compounds.

8. Tablet according to claim 6 or 7, **characterised in that** the dry binder is microcrystalline cellulose or a mixture of microcrystalline cellulose and a copolymer of vinylpyrrolidone and vinyl acetate.

9. Tablet according to one of claims 1 to 8, **characterised in that** it contains 0.2 to 80 wt.%, preferably 0.7 to 40 wt.% of a compound of formula I.

10. Tablet according to one of claims 1 to 9, **characterised in that** the weight ratio between the wetting agent and the active substance of formula I is in a range of about 1 : 200 to about 1 : 5, preferably between about 1 : 150 to about 1 : 10.

11. Tablet according to one of claims 5 to 10, **characterised in that** the weight ratio between lactose or mannitol and the active substance of formula I is in the range from about 20 : 1 to about 1 : 4, preferably about 12 : 1 to about 1 : 1.2.

12. Tablet according to one of claims 5 to 11, **characterised in that** the weight ratio between lactose and the active substance of formula I is in the range from about 4 : 1 to about 1 : 4, preferably about 1.8 : 1 to about 1 : 1.2.

13. Tablet according to one of claims 5 to 11, **characterised in that** the weight ratio between mannitol and the active substance of formula I is in a range of about 20:1 to about 1:2, more preferably in a range of about 15:1 to 3:1.

14. Tablet according to one of claims 6 to 13, **characterised in that** the amount by weight of the disintegrant based on the total mass of the tablet is in a range from about 0.5 to 10 wt.%.

15. Tablet according to one of claims 5 to 14, **characterised in that** the amount by weight of the flow agent, lubricant and separating agent based on the total mass of the tablet is in a range from about 0.1 to 5 wt.%.

16. Tablet according to one of claims 1 to 15, **characterised in that** it consists essentially of the following ingredients:
● a compound of formula I;
● microcrystalline cellulose;
● lactose or mannitol;
● optionally a copolymer of vinylpyrrolidone and vinyl acetate;
● sodium laurylsulphate;
● crosslinked polyvinylpyrrolidone or a crosslinked cellulosecarboxymethylether sodium salt, particularly croscarmellose sodium salt;
● magnesium stearate; and
● optionally a pharmaceutically acceptable colouring.

17. Process for preparing a tablet according to one of claims 1 to 16 by
(a) mixing a compound of formula I with a wetting agent and optionally other pharmacologically acceptable excipients, optionally in the presence of a volatile diluent;
(b) screening the mixture obtained;
(c) optionally adding a lubricant, and
(d) compressing with a suitable tablet press.

18. Use of a tablet according to one of claims 1 to 16 for preparing a pharmaceutical composition for the treatment or prevention of diseases in which LTB₄ antagonists can be used therapeutically or preventively.

19. Use of a tablet according to one of claims 1 to 16 for preparing a pharmaceutical composition for the treatment or prevention of arthritis, asthma, chronic obstructive pulmonary diseases, psoriasis, ulcerative colitis, Alzheimer's disease, shock, reperfusion damage/ischaemia, cystic fibrosis, atherosclerosis and multiple sclerosis.

## Revendications

1. Formulation médicamenteuse sous forme de comprimé contenant un antagoniste de LTB₄ de formule I 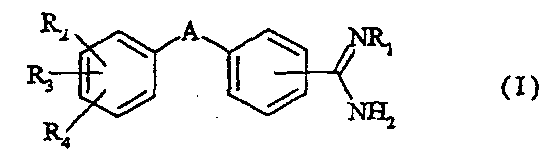 où
A représente un groupe de formule
-O-CₘH₂ₘ-O-(PHE)ₙ- (II)
où
m est un nombre entier de 2 à 6,
n est 0 ou 1,
PHE représente un groupe 1-4-phénylène éventuellement substitué par un ou deux groupes C₁-C₆-alkyle ;
ou
A représente un groupe de formule 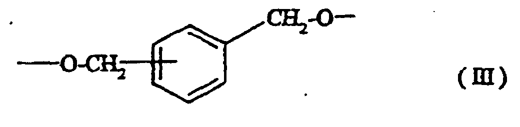
R₁ représente H, OH, CN, COR₁₀, COOR₁₀ ou CHO ;
R₂ représente H, Br, Cl, F, CF₃, CHF₂, OH, HSO₃-O-, C₁-C₆-alkyle, C₁-C₆-alcoxy, C₅-C₇-cycloalkyle, CONR₈R₉, aryle, O-aryle, CH₂-aryle, CR₅R₆-aryle ou C(CH₃)₂-R₇,
R₃ représente H, C₁-C₆-alkyle, C₁-C₆-alcoxy, OH, Cl ou F,
R₄ représente H ou C₁-C₆-alkyle,
R₅ représente C₁-C₄-alkyle, CF₃, CH₂OH, COOH ou COO(C₁-C₄-alkyle) ;
R₆ représente H, C₁-C₄-alkyle ou CF₃ ;
R₇ représente CH₂OH, COOH, COO(C₁-C₄-alkyle), CONR₈R₉ ou CH₂NR₈R₉ ;
R₈ représente H, C₁-C₆-alkyle, phényle, phényl-(C₁-C₆-alkyle), COR₁₀, COOR₁₀, CHO, CONH₂, CONHR₁₀, SO₂-(C₁-C₆-alkyle), SO₂-phényle, où le groupe phényle peut être substitué une ou deux fois par Cl, F, CF₃, C₁-C₄-alkyle, OH et/ou C₁-C₄-alcoxy ;
R₉ représente H ou C₁-C₆-alkyle ; ou
R₈ et R₉ pris ensemble représentent un groupe C₄-C₆-alkylène ;
R₁₀ représente C₁-C₆-alkyle, C₅-C₇-cycloalkyle, aryle, hétéroaryle, aralkyle ou hétéroaryl-(C₁-C₆-alkyle),
où les groupes aryle cités dans les groupements R₂ et R₁₀ représentent phényle ou naphtyle, les groupes hétéroaryle représentent pyrrole, pyrazole, imidazole, furanyle, thiényle, pyridine ou pyrimidine et peuvent être substitués chacun une ou plusieurs fois par Cl, F, CF₃, C₁-C₄-alkyle, OH, HSO₃-O- ou C₁-C₄-alcoxy, ainsi que ses sels d'addition d'acide et glycosides et 0-sulfates pharmacologiquement acceptables ;
et au moins un adjuvant pharmacologiquement acceptable, **caractérisée en ce que** le comprimé contient au moins un mouillant.

2. Comprimé selon la revendication 1 **caractérisé en ce que** l'antagoniste de LTB₄ est un composé choisi parmi les formules IA, IB et IC :

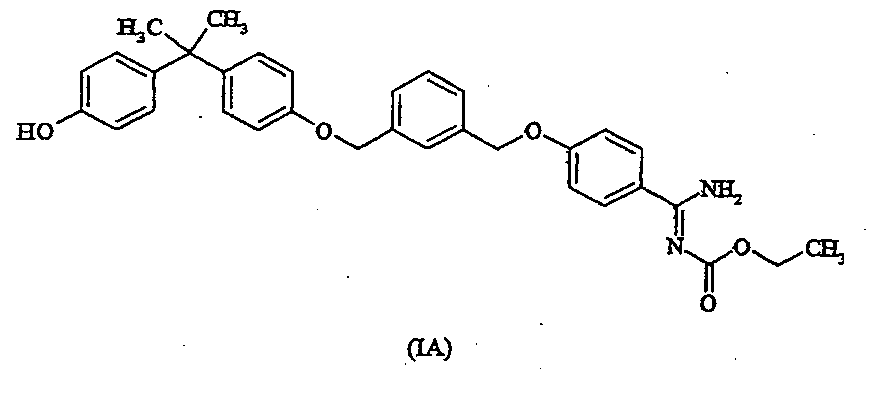

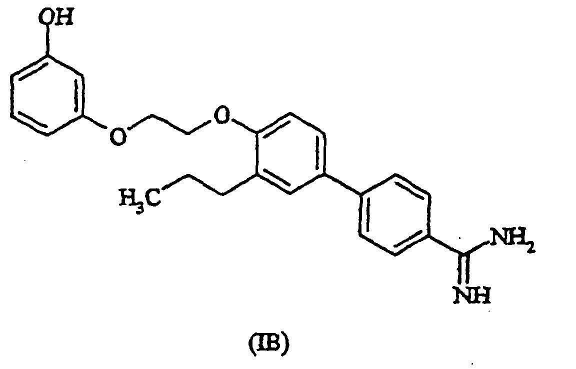

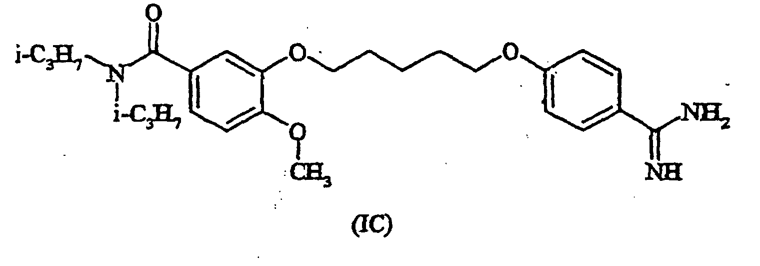

3. Comprimé selon l'une des revendications 1 ou 2 **caractérisé en ce que** le mouillant est une substance tensioactive anionique.

4. Comprimé selon l'une des revendications 1 à 3 **caractérisé en ce que** le mouillant est le laurylsulfate de sodium.

5. Comprimé selon l'une des revendications 1 à 4 **caractérisé en ce qu'**il contient du lactose ou du mannitol comme adjuvant.

6. Comprimé selon l'une des revendications 1 à 5 **caractérisé en ce qu'**il contient en outre au moins un liant sec, au moins un lubrifiant, au moins un désagrégeant et/ou au moins un agent de séparation et éventuellement un colorant.

7. Comprimé selon la revendication 6 **caractérisé en ce que** le liant sec est choisi dans le groupe consistant en la cellulose pulvérulente, la cellulose microcristalline, l'amidon, la polyvinylpyrrolidone, les copolymères de vinylpyrrolidone avec d'autres dérivés vinyliques, les dérivés cellulosiques et les mélanges de ces composés.

8. Comprimé selon la revendication 6 ou 7 **caractérisé en ce que** le liant sec est la cellulose microcristalline ou un mélange de cellulose microcristalline et d'un copolymère de vinylpyrrolidone et d'acétate de vinyle.

9. Comprimé selon l'une des revendications 1 à 8 **caractérisé en ce qu'**il contient 0,2 à 80 % en masse, de préférence 0,7 à 40 % en masse d'un composé de formule I.

10. Comprimé selon l'une des revendications 1 à 9 **caractérisé en ce que** le rapport massique entre le mouillant et le principe actif de formule I est dans un domaine d'environ 1 : 200 à environ 1 : 5, de préférence entre environ 1 : 150 et environ 1 : 10.

11. Comprimé selon l'une des revendications 5 à 10 **caractérisé en ce que** le rapport massique entre le lactose ou le mannitol et le principe actif de formule I est dans le domaine d'environ 20 : 1 à environ 1 : 4, de préférence d'environ 12 : 1 à environ 1 : 1,2.

12. Comprimé selon l'une des revendications 5 à 11 **caractérisé en ce que** le rapport massique entre le lactose et le principe actif de formule I est dans le domaine d'environ 4 : 1 à environ 1 : 4, de préférence d'environ 1,8 : 1 à environ 1 : 1,2.

13. Comprimé selon l'une des revendications 5 à 11 **caractérisé en ce que** le rapport massique entre le mannitol et le principe actif de formule I est dans un domaine d'environ 20 : 1 à environ 1 : 2, de manière particulièrement préférée dans un domaine d'environ 15 : 1 à 3 : 1.

14. Comprimé selon l'une des revendications 6 à 13 **caractérisé en ce que** la proportion massique de désagrégeant par rapport à la masse totale du comprimé est située dans un domaine d'environ 0,5 à 10 % en masse.

15. Comprimé selon l'une des revendications 5 à 14 **caractérisé en ce que** la proportion massique d'agent d'écoulement, de lubrifiant et d'agent de séparation par rapport à la masse totale du comprimé est située dans un domaine d'environ 0,1 à 5 % en masse.

16. Comprimé selon l'une des revendications 1 à 15 **caractérisé en ce qu'**il consiste essentiellement en les constituants suivants :
- un composé de formule I ;
- de la cellulose microcristalline ;
- du lactose ou du mannitol ;
- éventuellement un copolymère de vinylpyrrolidone et d'acétate de vinyle ;
- du laurylsulfate de sodium ;
- de la polyvinylpyrrolidone réticulée ou un sel de sodium de carboxyméthyléther de cellulose réticulé, en particulier le sel de sodium de croscarmellose ;
- du stéarate de magnésium ; et
- éventuellement un colorant pharmaceutiquement acceptable.

17. Procédé de production d'un comprimé selon l'une des revendications 1 à 16 par
(a) mélange d'un composé de formule I avec un mouillant et éventuellement d'autres adjuvants pharmacologiquement acceptables éventuellement en présence d'un diluant volatil ;
(b) tamisage du mélange obtenu ;
(c) éventuellement addition d'un lubrifiant, et
(d) compression avec une presse à comprimés appropriée.

18. Utilisation d'un comprimé selon l'une des revendications 1 à 16 pour la production d'un médicament pour le traitement ou la prévention de maladies dans lesquelles des antagonistes de LTB₄ peuvent être utilisés thérapeutiquement ou préventivement.

19. Utilisation d'un comprimé selon l'une des revendications 1 à 16 pour la production d'un médicament pour le traitement ou la prévention de l'arthrite, de l'asthme, des maladies pulmonaires obstructives chroniques, du psoriasis, de la colite ulcéreuse, de la maladie d'Alzheimer, du choc, des lésions de reperfusion/ischémies, de la fibrose kystique, de l'athérosclérose et de la sclérose en plaques.
